# EUROPEAN PATENT APPLICATION

(11) **EP 4 312 185 A1**
(43) Date of publication of application: **31.01.2024**
(21) Application number: 22315173.9
(22) Date of filing: 27.07.2022
(51) Int. Cl.: G06T 7/00, A61B 8/08, G06T 7/33

(54) **METHOD AND SYSTEM OF LINKING ULTRASOUND IMAGE DATA ASSOCIATED WITH A MEDIUM WITH OTHER IMAGE DATA ASSOCIATED WITH THE MEDIUM**

(71) Applicant: SUPERSONIC IMAGINE, 13290 Aix-en-Provence (FR)
(72) Inventor: FRASCHINI, Christophe, 13290 Aix-en-Provence (FR); ZHANG, Bo, 13290 Aix-en-Provence (FR)
(74) Representative: Paustian & Partner Patentanwälte mbB

(57) **Abstract**

The disclosure relates to a method of linking ultrasound image data associated with a medium with other image data associated with the medium. The method comprises: estimating deforma'bility properties of the medium based on the ultrasound data and linking the ultrasound image data with the other image data based on the deformability properties.

## Description

### FIELD OF THE DISCLOSURE

The present technology relates to methods and systems of linking ultrasound image data associated with a medium with other image data associated with the medium, in particular for medical imaging. In particular, the method is suitable for processing signal data of a medium.

### BACKGROUND

It is known to use a plurality of transducer elements or transceivers (for example arranged as an array) for communication, imaging or scanning purposes, for example in the field of medical imaging, radar, sonar, seismology, wireless communications, radio astronomy, acoustics and biomedicine. One example comprises ultrasound imaging.

An aim of ultrasound imaging is to estimate the medium reflectivity. In a conventional ultrasound imaging method, an ultrasound transducer device (also referred to as an ultrasound probe) with a set of ultrasound transducer elements may be used. In the method, one or multiple transducers are used to transmit one or successively several ultrasound beam into a medium, corresponding to a transmission operation. Then, in a reception operation, a set of backscattered echo signals are received from the medium by the set of transducer elements. In particular, each of the transducer elements converts a received echo signal into for example an electrical signal. The signal may further be processed by the ultrasound system and/or by the probe. For example, they may be amplified, filtered digitalized and/or a signal conditioning operation may be carried out. The transducer elements may be arranged as a transducer array.

Other imaging methods than ultrasound imaging exist, for example methods using optical and/or x-ray imaging.

### SUMMARY OF THE DISCLOSURE

Currently, it remains desirable to provide a method and system of linking ultrasound image data associated with a medium with other image data associated with the medium, which advantageously is reliable and robust.

Such a method for example may be used in the context of breast examinations: Mammography or tomosynthesis screenings enable to detect early cancers and microcalcifications, and it has proven a mortality reduction of more than 30%. However, its efficacy decreases in presence of dense breasts, and it is not always suitable during interventional procedure like biopsy.

When a lesion with a potential risk has been identified with mammography or tomosynthesis, the patient usually goes to ultrasound exam for diagnosis. Since the breast is squeezed between two plaques during mammography or tomosynthesis exam, while during the ultrasound exam, the breast is not physically constrained, it may be difficult for the person who is performing the ultrasound exam to ensure that the lesion observed with ultrasound is actually the one that has been identified. Both the breast and the lesion are deformed during mammography and/or during tomosynthesis and/or during tomography. As a result, it may be difficult to predict the location of a lesion identified with the mammography/the tomosynthesis during an ultrasound exam. Furthermore, the shape of a lesion may be different as well.

Therefore, a method of linking ultrasound image data associated with a medium with other image data associated with the medium is provided, and the method comprises: estimating deformability properties of the medium based on the ultrasound data and linking the ultrasound image data with the other image data based on the deformability properties.

The terms "associated with" and "represented by" may be used synonymously throughout this disclosure unless denoted differently.

The present disclosure may for example allow to link lesions identified in a medium for example based on mammography or tomosynthesis with lesions in the medium identified based on ultrasound imaging. The linking for example may be defined by the system of the present disclosure and/or it for example may be automated.

The present disclosure may for example provide an increased linking efficacy as it may ensure, optionally with a confidence level, that a lesion detected and identified based on mammography and/or tomosynthesis data may be retrieved based on ultrasound data. It may be possible to characterize the lesion based on ultrasound image data, combining benefits from the other imaging method, for example mammography or tomosynthesis, and ultrasound. If a biopsy is needed, then ultrasound guided biopsy may be performed, optionally with a confidence level, at the location identified for example based on the mammography image data and/or the tomosynthesis image data. All these operations do not depend on the skills of the person who is scanning. The optional confidence level may for example indicate to the user how confident the guidance is, i.e. whether the user may more or less trust on the provided guidance.

The present disclosure may for example allow for an increased linking efficiency because for example an ultrasound lesion finding procedure may be automated and reproducible.

Deformability properties may comprise three-dimensional (3D) deformability properties and/or 2D deformability properties. 3D deformability properties may be beneficial for establishing a realistically deformed 3D model of the medium and/or for realistically simulating a deformation of the medium and/or a deformed medium. Deformability properties may allow to build a model of the medium, for example a parametric model, for example a 3D parametric model.

The terms "deformation" and "deformation state" may be used synonymously throughout this disclosure unless denoted differently.

Deformability properties may allow to simulate a deformation of the medium and/or a deformed medium. A deformation of the medium and/or a deformed medium may relate to a deformation of the medium compared to the medium as associated with/represented by image data, for example the ultrasound image data and/or the other image data and/or third image data. Simulating a deformed medium may be based on the model.

Linking the ultrasound image data with the other image data based on the deformability properties may comprise matching and/or mapping and/or correlating the ultrasound image data with the other image data based on the deformability properties. Linking the ultrasound image data with the other image data based on the deformability properties may comprise matching and/or mapping and/or correlating the ultrasound image data with the other image data based on the deformability properties, for example by linking a simulated deformed medium/a simulation of a deformation of the medium with the other image data. The linking, matching, mapping, and/or correlating may comprise one or more confidence levels, that may for example score the linking, matching, mapping, and/or correlating with a respective confidence level. The confidence levels may be preselected in a known range of values, that may be displayed to the end user.

Linking the ultrasound image data with the other image data based on the deformability properties may comprise: linking a simulated deformed medium/a simulation of a deformation of the medium, for example a simulation of a deformation of the medium compared to the medium as associated with the ultrasound image data, with the other image data. The simulation for example may be based on the ultrasound image data.

Linking the ultrasound image data with the other image data based on the deformability properties may comprise: linking the ultrasound image data with a simulated deformed medium/a simulation of a deformation of the medium, for example a simulation of a deformation of the medium compared to the medium as associated with the other image data. The simulation for example may be based on the other image data.

Linking the ultrasound image data with the other image data may allow to combine information about the medium obtained from image data. Thus, more information and/or complimentary information can be obtained, and advantages of both methods can be obtained. For example, the medium as associated with the ultrasound image data may be not deformed and/or may in a similar deformation state compared to the medium when subject to further medical treatment, for example during surgery, which may facilitate the further medical treatment, for example surgery. The other imaging method, for example mammography and/or tomosynthesis, may be a standard method for performing a specific task, for example searching for lesions.

Linking the ultrasound image data with the other image data may be understood as defining a relationship between the ultrasound image data and the other image data.

Linking the ultrasound image data with the other image may be based on tracking schemes. For example, a block-matching algorithm may be used. A block-matching algorithm is for example disclosed in the patent application EP 3 771 927 A1 of the applicant. For example, a pixel tracking algorithm may be used.

The other image data may comprise at least one of: image data originating from another source than an ultrasound imaging source, X-ray image data, mammography and/or tomosynthesis and/or tomography image data, Magnetic Resonance (MR) image data, image data originating from an optical imaging device, and other ultrasound image data. The other image data may comprise image data that are image data associated with the interior of the medium and/or interior image data.

Image data originating from an optical device may for example comprise diffuse optical tomography image data.

The ultrasound data may comprise and/or may be 3D ultrasound image data, and/or the ultrasound data may comprise at least one of: shear wave elasticity (SWE) data, non-linear shear wave elasticity (NL-SWE) data, B-mode data.

The ultrasound image data, for example the 3D ultrasound image data, may be obtained using a predefined ultrasound imaging process and/or using a 3D transducer and/or using a handheld device which optionally is combined with navigation. The ultrasound image data, for example the 3D ultrasound image data, may be obtained using a regular 1D array transducer, optionally coupled with a navigation system, and/or an automated breast system, for example with 1D array transducer and mechanical sweeping, and/or a dedicated matrix array transducer.

Shear wave elasticity data may be obtained in combination with B-mode imaging. When a shear wave elasticity ultrasound imaging method is applied, it is not necessary to deform the medium for estimating elasticity properties of the medium.

Non-linear shear wave elasticity data may be obtained in combination with B-mode image. Non-linear shear wave elasticity may require a relatively slight deformation of the deformation or one or several deformation steps. Based on the obtained non-linear shear wave elasticity data, different deformation states of the medium may be estimated, including relatively strong deformations.

One example of obtaining non-linear shear wave elasticity data is for example disclosed in the patent application FR1914432A of the applicant. A temporal succession of shear wave elasticity data may be collected from a medium while a successively changing deformation is applied to the medium. It is possible to quantify the non-linear elasticity of the medium as a function of on the temporal succession of shear wave elasticity data and the deformation variation.

B-mode data may be used to assess the geometry of the medium to generate a model. For example, a (geometrical/physical) model of the medium may be build based on finite element modelling and/or based on B-mode data. The (geometrical) model may mesh the image data.

Attenuation properties of the medium may be estimated based on the ultrasound data. Linking the ultrasound image data with the other image data additionally may be based on the estimated attenuation properties. In other words: Attenuation properties estimated and/or derived from ultrasound data may be used to improve the linking accuracy.

The medium as associated with and/or represented by the other image data may be in a different deformation state compared to the medium as associated with and/or represented by the ultrasound image data.

The different deformation states may be related to/caused by a force and/or an external force acting on the medium. The different deformation states may be related to/caused by a pressure and/or an external pressure acting on the medium.

The medium as associated with and/or represented by the ultrasound image data may be deformed and/or may be not deformed, i.e. subject to an external force/pressure or to no external force/pressure. For example, during acquisition of non-linear shear wave elasticity image data, the medium may be deformed and the state of deformation may vary. In another example, during acquisition of shear wave elasticity image data, the medium is not required to be deformed and/or is only slightly deformed by an ultrasound probe on it.

Linking the ultrasound image data with the other image data based on the deformability properties may allow to combine information from both image data in a reliable and reproducible way that is not hampered by a deformation of the medium as represented by/associated with the other image data and/or the ultrasound image data.

For example, the other image data may comprise mammography image data. During a mammography examination, usually the patient is standing and the breast is compressed between two plaques, while during an ultrasound examination the patient usually is lying and there is usually no (major) external compression (except for example from a different influence of gravity etc.).

The other image data may comprise tomosynthesis image data. Also during a tomosynthesis examination, the breast of the patient is usually deformed by external forces/by external pressures, including plaques.

During the ultrasound examination on for example a breast, a specific region of the breast may be a region of interest (ROI). During the ultrasound examination on for example a breast, a specific portion of the breast may correspond to a region of interest (ROI). The deformability/elasticity/stiffness properties of the breast may be estimated using another region than the ROI/another portion than that associated to the ROI, and the ROI/the associated portion may be imaged afterwards using the evaluated compression.

Linking the ultrasound image data with the other image data may comprise: linking the ultrasound image data with third image data, and linking the other image data with the third image data.

The third image data may be for example MR image data, and the other image data may be for example X-ray image data. Linking the ultrasound image data with the other image data based on the deformability properties may comprise: linking the ultrasound image data with the third image data, for example the MR image data, and linking the other image data, for example the X-ray image data, with the third image data, for example the MR image data.

Linking the ultrasound image data with third image data may be based on the deformability properties. Linking the other image data with the third image data may be based on the deformability properties.

Linking the ultrasound image data and/or the other image data with third image data may comprise: Linking a simulated deformed medium/a simulation of a deformation of the medium, for example a simulation of a deformation of the medium compared to the medium as associated with the ultrasound image data and/or the other image data, with the third image data. The simulated deformed medium/the simulation of a deformation of the medium may be simulated based on the deformability properties. The simulated deformed medium/the simulation of a deformation of the medium may be simulated based on the respective image data, for example the ultrasound image data and/or the third image data and/or the other image data. The simulated deformed medium/the simulation of a deformation of the medium may be simulated based on the respective imaging methods, for example the ultrasound imaging method and/or the third imaging method and/or the other imaging method, for example based on a typical deformation for the respective imaging methods, for example the ultrasound imaging method and/or the third imaging method and/or the other imaging method, for example based on a typical external force applied to the medium during the respective imaging method, for example the ultrasound imaging method and/or the third imaging method and/or the other imaging method.

Linking the ultrasound image data/the other image data with third image data may comprise: Linking the ultrasound image data/the other image data with a simulated deformed medium/a simulation of a deformation of the medium, for example a simulation of a deformation of the medium compared to the medium as associated with the third image data. The simulated deformed medium/the simulation of a deformation of the medium may be simulated based on the deformability properties. The simulated deformed medium/the simulation of a deformation of the medium may be simulated based on the imaging method, for example the third imaging method, for example based on a typical deformation for the imaging method, for example the third imaging method, for example based on a typical external force applied to the medium during the imaging method, for example the third imaging method.

Linking the ultrasound image data/the other image data with third image data may comprise: Linking a simulated deformed medium/a simulation of a deformation, the simulation of a deformation being based on the ultrasound image data/the other image data, for example a simulation of a deformation of the medium compared to the medium as associated with the ultrasound image data and/or the other image data, with another simulated deformed medium/another simulation of a deformation, the another simulation of a deformation being based on the third image data, for example a simulation of a deformation of the medium compared to the medium as associated with the third image data. The respective simulations may for example each be based on the deformability properties.

The third image data, for example the MR image data, may provide a reference for linking the ultrasound image data with the other image data. The third image data, for example the MR image data, may be able to link the ultrasound image data with the other image data, for example the X-ray image data. This may especially be beneficial in case a deformation state of the medium as associated with/represented by the ultrasound image data and/or the X-ray image data may have an unknown and/or imprecisely/inaccurately known deformation state and in case the deformation state of the medium as associated with/represented by the third image data, for example the MR image data, is known. The deformation state of the medium as associated with the third image data may be a reference deformation state. Then, for each linking operation there may be only one unknown deformation condition/the deformation state of only one image data may be unknown, as opposed to having unknown deformation states of both image data linked with each other.

The medium may comprise human breast tissue.

The medium may for example comprise animal tissue, human tissue, human breast tissue, a whole human breast, musculoskeletal material, a spatial region in the upper body, a portion of the upper body, a spatial region in the abdomen, a portion of the abdomen, a deformable medium, non-human tissue, non-human material, and/or a phantom.

Estimating deformability properties may comprise estimating at least one of: elasticity properties of the medium, non-linear elasticity properties of the medium, stiffness properties of the medium, geometrical properties of the medium, structural and/or granularity properties of the medium, density properties of the medium, absorption properties of the medium.

Estimating deformability properties may comprise estimating transparency properties and/or reflection properties and/or absorption properties. Estimating deformability properties may comprise estimating viscoelasticity of the considered medium.

Elasticity properties of the medium may comprise Youngs modulus. Non-linear elasticity properties of the medium may comprise non-linear shear modulus.

The deformability properties may be non-uniform in the medium. Estimating deformability properties of the medium may comprise estimating non-uniform deformability properties of the medium.

Elasticity properties of the medium may be determined for example based on shear wave elasticity data. Non-linear elasticity properties of the medium may be determined for example based on non-linear shear wave elasticity data. Stiffness properties of the medium may be determined for example based on shear wave elasticity data and/or non-linear shear wave elasticity data.

Geometrical properties of the medium for example may be determined based on B-mode image data. Geometrical properties for example may be determined based on data of an optical sensor, for example a camera. Geometrical properties may for example comprise exterior geometrical properties. Exterior geometrical properties may be determined for example by an optical sensor, for example a camera. Geometrical properties for example may be determined using a localization tracker providing localization information of an ultrasound transducer/ultrasound probe used for obtaining the ultrasound data. Geometrical properties for example may be determined based on markers present in the medium, for example geometrical landmarks. The term marker may comprise implanted markers. Markers in the medium may enable a better estimation of the deformation/the deformed medium and/or a better simulation thereof.

Determining structural and/or granularity properties of the medium may comprise: Performing volume segmentation of the medium, for example ultrasound volume segmentation. Volume separation may comprise separation of adipose and glandular tissue. Structural and/or granularity properties of the medium may be determined based on B-mode image data.

Density properties of the medium may be determined for example based on speed of sound estimations.

Absorption properties of the medium may be determined for example based on the ultrasound data, e.g. based on attenuation estimation performed on the ultrasound data.

Deformability properties may be estimated based on at least one of the following methods: Finite Element modelling (FEM), Finite Element modelling using boundaries, estimating Youngs modulus, estimating non-linear shear modulus, modelling using Active Cubes and/or marching cubes, Boundary Element Modelling, and/or a predefined registration method.

Estimating deformability properties may comprises and/or may be based on at least one of: Finite Element modelling (FEM), optionally FEM using boundaries, estimating Youngs modulus, estimating non-linear shear modulus, modelling using Active Cubes and/or marching cubes, Boundary Element Modelling, and/or a predefined registration method.

Finite Element modelling for example may be based on/linked to B-mode image data. Youngs modulus for example may be determined/obtained/estimated based on shear wave elasticity data. Non-linear shear modulus for example may be determined/obtained/estimated based on non-linear shear wave elasticity data.

A predefined registration method for example may be used in case deformability properties are derived from two data sets of marker positions.

Linking the ultrasound image data with the other image data may comprise: simulating a deformed medium based on the deformability properties, such that the simulated deformed medium matches with the deformation state of the medium as associated with the other image data.

A deformation of the medium and/or a deformed medium may relate to a deformation of the medium compared to the medium as associated with/represented by respective image data, for example the ultrasound image data and/or the other image data and/or the third image data.

Linking the ultrasound image data with the other image data may comprise: simulating a deformed medium such that the simulated deformed medium corresponds to and/or relates to the deformation state of the medium as associated with the other image data. Linking the ultrasound image data with the other image data may comprise: simulating a deformed medium according to the deformation state of the medium as associated with the other image data. Linking the ultrasound image data with the other image data may comprise: simulating a deformed medium such that the simulated deformed medium is associated with the deformation state of the medium as associated with the other image data.

Linking the ultrasound image data with the other image data may comprise: Linking a simulated deformed medium, for example a simulation of a deformation of the medium compared to the medium as associated with the ultrasound image data, with the other image data. Linking the ultrasound image data with the other image data may comprise: Linking the ultrasound image data with the simulated deformed medium, for example a simulation of a deformation of the medium compared to the medium as associated with the other image data.

The simulated deformed medium may be simulated based on the deformability properties and/or based on the ultrasound image data and/or based on the other image data.

Simulating a deformed medium may comprise a predefined mathematical operation.

Simulating a deformed medium may comprise building a model of the medium, for example a parametric model of the medium, for example a 3D parametric model. The parametric model may be based on the deformability properties of the medium. Simulating a deformed medium may be based on the deformability properties of the medium.

Simulating a deformed medium may comprise and/or may start with a coarse simulation of a deformation in the model. The coarse simulation may be based on/may be guided by the deformability properties to simulate a realistic deformation of the medium. Simulating a deformed medium may further comprise: Refining the coarse simulation such that the simulation satisfies/corresponds to the deformability properties in every region of the medium/in every portion of the medium. Optionally, the simulation may be compared with the estimated deformability properties of the medium and it may be checked whether the estimated deformability properties of the medium are satisfied/corresponded to in the simulation. The estimated deformability properties may for example comprise: elasticity properties of the medium, non-linear elasticity properties of the medium, stiffness properties of the medium, geometrical properties of the medium, structural and/or granularity properties of the medium, and/or density properties of the medium.

Markers in the medium may be used for estimating the deformation state of the medium as associated with/represented by the mammography image and evaluating whether this level of deformation is reached in the simulated deformed medium.

It is also possible to use the mammography image data to evaluate whether the level of deformation of the medium as associated with/represented by the mammography image data is reached in the simulated deformed medium.

Simulating a deformed medium such that the simulated deformed medium matches with the deformation state of the medium as associated with the other image data may comprise: Assuming a force corresponding to an external force applied to the medium as associated with/represented by for example the other image data. Simulating a deformed medium such that the simulated deformed medium matches with the deformation state of the medium as associated with the other image data may comprise: Assuming a pressure corresponding to an external pressure applied to the medium as associated with/represented by for example the other image data.

Linking the ultrasound image data with the other image data based on the deformability properties may further be based on the simulated deformed medium.

Simulating the deformed medium may further be based on the other image data and/or simulating the deformed medium may use the other image data, for example in a control loop.

Simulating the deformed medium based on the other image data and/or using the other image data in a control loop may allow to obtain a better/more accurate/more precise simulated deformed medium and/or it may allow to improve linking the ultrasound image data with the other image data.

Linking the ultrasound image data with the other image data may comprise: determining a first subset of the ultrasound image data associated with/corresponding to a first portion of the medium/first region in the medium, determining a second subset of the other image data associated with/corresponding to second portion of the medium/a second region in the medium, and linking the second subset with the first subset, optionally based on the simulated deformed medium.

The first portion of the medium and the second portion of the medium may correspond to spatial regions in the medium. The first portion of the medium and the second portion of the medium may correspond to lesions in the medium. The first portion of the medium and the second portion of the medium may be determined based on manual input and/or based on using a predefined computer-implemented algorithm, for example a regular automated algorithm and/or an artificial intelligence (AI)-based algorithm, for example it may involve using a neural network, a convolutional neural network and/or the like. For example, a tumor recognition technique may be used. The first portion of the medium and/or the second portion of the medium may be determined based on pattern recognition schemes.

Linking the second subset with the first subset may for example allow to link a lesion identified in a mammography and/or a tomosynthesis examination with a lesion identified in an ultrasound examination. The first subset and/or the second subset may for example comprise a lesion and/or conspicuous tissue. The lesion identified in the ultrasound image data and the lesion identified in the other image data for example may be the same lesion. Linking the second subset with the first subset may for example allow to link the location of a lesion identified in a mammography and/or a tomosynthesis examination with the location of a lesion identified in an ultrasound examination, for example with the same lesion.

The first portion of the medium and the second portion of the medium may be labeled.

The second subset may be linked with the first subset and/or the first subset may be linked with the second subset.

The first region in the medium/the first portion of the medium may overlap with and/or may be identical to the second region in the medium/the second portion of the medium. In particular, the first region in the medium/the first portion of the medium and the second region in the medium/the second portion of the medium may comprise identical tissue.

Determining the first subset and/or determining the second subset and/or linking the second subset with the first subset may be based on tracking schemes. For example, for linking the second subset with the first subset, a block-matching algorithm may be used. A block-matching algorithm is for example disclosed in the patent application US16/941,865 of the applicant. For example, a pixel tracking algorithm may be used.

Linking the ultrasound image data with the other image data may comprise: Determining/identifying ultrasound image data and other image data that each represent the same and/or a similar portion of the medium and/or the same and/or a similar region in the medium.

Some imaging methods may lead to generate image data that correspond to 3D images, for example 3D ultrasound and tomography, while some imaging methods are able to provide image data that correspond to 2D projections of a 3D medium, for example mammography and tomosynthesis, for example tomosynthesis slices. For example, if the other image data comprise mammography image data and/or tomosynthesis image data, linking the ultrasound image data with the other image data may for example allow to link ultrasound 3D data to 2D mammography data.

This disclosure advantageously provides easy and reproducible methods that allow to link image data of different dimensions with each other.

Linking the ultrasound image data with the other image data may be based on the simulated deformed medium. Linking the second subset with the first subset may be based on the simulated deformed medium, for example linking the second subset with the first subset may comprise linking the second subset with a simulated deformed first subset and/or a first subset in a simulated deformed medium. Linking the second subset with the first subset may be based on the simulated deformed medium, for example linking the second subset with the first subset may comprise linking a deformed second subset and/or a second subset in a simulated deformed medium with the first subset.

The first portion of the medium may be distinguished from the surrounding medium based on at least one of: elasticity properties of the medium, non-linear elasticity properties of the medium, an image segmentation and/or classification method using a predefined computer-implemented algorithm, and/or the second portion of the medium may be distinguished from the surrounding medium based on an image segmentation and/or classification method using a predefined computer-implemented algorithm.

The first portion of the medium may be distinguished from the surrounding medium for example based on transparency properties and/or reflection properties and/or viscoelastic properties, e.g. by the viscoelasticity of the considered medium.

The first portion of the medium and/or the second portion of the medium may comprise at least one of: a benign or malignant tumor, a lesion, suspicious tissue, a tumor candidate, a marker, a breast cancer.

The predefined computer-implemented algorithm for example may be AI-based, for example it may involve using a neural network, a convolutional neural network or the like. For example, a tumor recognition technique may be used.

The method may optionally comprise determining a deformability properties confidence and/or a deformability properties quality. The method may further comprise: Determining a linking confidence of linking the ultrasound image data with the other image data. Determining a linking confidence may be based on at least one of: the deformability properties, the deformability properties confidence.

A linking confidence may be for example a confidence index.

The deformability properties confidence for example may be a measure for the quality and/or the confidence of the estimation of the deformability properties. The deformability properties confidence for example may be error bars/uncertainty bars at pre-defined confidence levels. A lower deformability properties confidence may for example lead to a lower linking confidence. Further, it for example may be easier to link the ultrasound image data with the other image data if deformability properties are within specific value ranges. In this way for example, a linking confidence may be based on the deformability properties or it may be impacted by the deformability properties.

The deformability properties confidence for example may be related to differences between the simulated deformed medium and the medium as represented by/associated with and/or reconstructed from the other image data. The deformability properties confidence for example may be determined based on differences between the simulated deformed medium and the medium as represented by and/or reconstructed from the other image data.

The method may further comprise determining a linking confidence of linking the second subset with the first subset. Determining a linking confidence of linking the second subset with the first subset may be based on at least one of: a spatial distance of the first portion and of the second portion/between the first portion and the second portion, a spatial distance of the first portion in a simulated deformed medium and the second portion/between the first portion in a simulated deformed medium and the second portion, a spatial overlap between the first portion and the second portion, a spatial overlap of the first portion in a simulated deformed medium and the second portion, a contour and/or a shape and/or a size of the first and/or the second portion, a contour and/or a shape and/or a size of the first portion in a simulated deformed medium and/or of the second portion, the deformability properties, the deformability properties confidence.

Determining a linking confidence of linking the second subset with the first subset may be based on at least one of: a spatial distance of the first portion and of the second portion in a simulated deformed medium/between the first portion and the second portion in a simulated deformed medium, a spatial overlap between the first portion and the second portion, a spatial overlap of the first portion and of the second portion in a simulated deformed medium, a contour and/or a shape and/or a size of the first and/or the second portion, a contour and/or a shape and/or a size of the first portion and/or of the second portion in a simulated deformed medium.

A linking confidence of linking the second subset with the first subset for example may be a score and/or a scoring of linking the second subset with the first subset.

For example, if contours and/or shapes and/or sizes of the first portion and the second portion are very similar, the linking confidence may be higher. Determining a linking confidence of linking the second subset with the first subset may be based on a Bayesian method and/or a conditional method, and for example based on the deformability properties confidence and/or differences between the simulated deformed medium and the medium as represented by and/or reconstructed from the other image data.

Determining a linking confidence of linking the second subset with the first subset may be performed using a computer-implemented algorithm. The computer-implemented algorithm may be AI-based, it may comprise use of learning schemes, for example deep learning and/or machine learning, and/or an artificial intelligence and/or a neural network and/or a convolutional neural network and/or the like. The computer-implemented algorithm may further be based on manual input by a user and/or may be performed in an automated way. The computer-implemented algorithm may be for example Kernel-based, it may involve for example contour tracking, Kalman filtering, particle filtering. The computer-implemented algorithm may be for example automated.

Determining the linking confidence of linking the ultrasound image data with the other image data may optionally be based on determining the linking confidence of linking the second subset with the first subset.

Simulating a deformed medium and/or determining a first subset and/or determining a second subset and/or linking the second subset with the first subset and/or determining a linking confidence of linking the ultrasound image data with the other image data and/or determining a linking confidence of linking the second subset with the first subset may be performed using/may be based on a computer-implemented algorithm.

Determining a deformability properties confidence may be performed using a computer-implemented algorithm.

The terms "confidence" and "quality" may be used synonymously throughout this disclosure unless denoted differently.

The computer-implemented algorithm for example may be AI-based, it may comprise use of learning schemes, for example deep learning and/or machine learning, and/or an artificial intelligence and/or a neural network and/or a convolutional neural network and/or the like. The computer-implemented algorithm may further be based on manual input by a user and/or may be performed in an automated way. The computer-implemented algorithm may be for example Kernel-based, it may involve for example contour tracking, Kalman filtering, particle filtering. The computer-implemented algorithm may be for example automated.

The computer-implemented algorithm may comprise applying tracking schemes for linking the first subset with the second subset and/or for determining a linking confidence. The applied tracking schemes may involve contour tracking, Kalman filtering, particle filtering, learning schemes, for example deep learning.

Also a plurality of first subsets associated with/corresponding to a plurality of first portions of the medium/a plurality of first regions in the medium and/or a plurality of second subsets associated with/corresponding to a plurality of second portions of the medium/a plurality of second regions in the medium may be determined and may be linked with each other, for example using a loss function, and for example corresponding linking confidences may be determined. For example, several loops of linking first subsets with second subsets may be performed and matrices with linking confidences may be built.

The present disclosure may refer to a computer program comprising computer-readable instructions which when executed by a data processing system cause the data processing system to carry out a method according to any examples of the present disclosure.

The present disclosure may refer to a system for linking ultrasound image data associated with a medium with other image data associated with the medium. The system may comprise a processing unit configured to: estimate deformability properties of the medium based on the ultrasound data, and link the ultrasound image data with the other image data based on the deformability properties. The system may furthermore be configured to perform any of the method features or operations described above.

The present disclosure may also refer to a system for linking ultrasound image data associated with a medium with other image data associated with the medium. The system may comprise a processing unit and a memory coupled to the processing unit, the memory comprising computer executable instructions that, when executed by the processing unit, perform a method comprising configured to: estimating deformability properties of the medium based on the ultrasound data, and linking the ultrasound image data with the other image data based on the deformability properties.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification , illustrate examples of the disclosure and together with the description, and serve to support and illustrate the principles thereof.

### BRIEF DESCRIPTION OF THE DRAWINGS

Fig. 1 shows an exemplary embodiment of a method according to embodiments of the present disclosure.
Fig. 2 illustrates the relation between a parametric model of the medium, a simulated deformed medium, and other image data.
Fig. 3 shows another exemplary embodiment of a method according to embodiments of the present disclosure.
Fig. 4 shows yet another exemplary embodiment of a method according to embodiments of the present disclosure.

### DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

WO 2017/205386 A1 of the applicant discloses a method for imaging that includes acquiring surface image data for a target using a first imaging modality. A visual representation of the target based on the surface image data is generated. Internal image data for the target is acquired using a second imaging modality. During acquisition of the internal image data, the visual representation of the target based on the acquired internal image data is updated.

This method relates to surface image data and internal image data.

WO 2019/035064 A1 of the applicant discloses techniques for breast imaging patient motion compensation. An imaging system may include an imaging detector to capture an image of human tissue and a compression paddle situated apart from the imaging detector to compress the human tissue between the compression paddle and the imaging detector. A force sensor may generate a force signal indicating a measure of force applied superior to the human tissue. A movement detection circuit may filter a movement signal from the force signal indicating a measure of movement of the compressed human tissue. A movement analysis module may determine that the movement signal is beyond a movement threshold. An image correction module to perform a corrective action based upon the determination that the movement signal is beyond a movement threshold. Other embodiments are described and claimed.

This method relates to performing imaging correction based on input from a movement detection circuit.

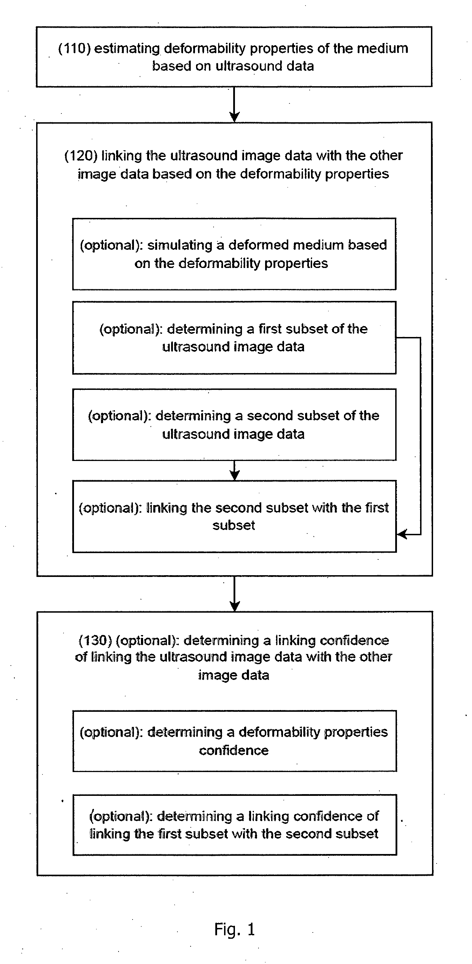

Fig. 1 shows an exemplary embodiment of a method according to embodiments of the present disclosure. The method may be carried out by a system comprising a processing unit configured to: estimate deformability properties of the medium based on the ultrasound data and link the ultrasound image data with the other image data based on the deformability properties. The system may furthermore be configured to perform any of the method features or operations described in this disclosure.

The method may be an ultrasound method carried out by an ultrasound system. Possible ultrasound methods comprise B-mode imaging, shear wave elastography imaging (such as ShearWave^{®} mode developed by the applicant), Doppler imaging, CEUS imagine, ultrafast^{™} Doppler imaging or angio mode named under Angio PL.U.S ^{™} ultrasound imaging or any other ultrasound imaging mode. Accordingly, different acquisition modes may be used to obtain ultrasound image data. The method may be part of any of the above-mentioned methods or may be combined with any of these methods.

In an operation (110), deformability properties of the medium are estimated based on the ultrasound data.

Deformability properties may comprise three-dimensional (3D) deformability properties and/or 2D deformability properties. 3D deformability properties may be beneficial for establishing a realistically deformed 3D model of the medium and/or for realistically simulating a deformation of the medium and/or a deformed medium. Deformability properties may allow to build a model of the medium, for example a parametric model, for example a 3D parametric model.

Deformability properties may allow to simulate a deformation of the medium and/or a deformed medium. A deformation of the medium and/or a deformed medium may relate to a deformation of the medium compared to the medium as associated with/represented by image data, for example by the ultrasound image data and/or the other image data and/or the third image data. Simulating a deformed medium may be based on the model.

In an operation (120), the ultrasound image data are linked with the other image data based on the deformability properties.

Linking the ultrasound image data with the other image data based on the deformability properties may comprise matching and/or mapping and/or correlating the ultrasound image data with the other image data based on the deformability properties, for example by linking a simulated deformed medium/a simulation of a deformation of the medium with the other image data. The linking, matching, mapping, and/or correlating may comprise one or more confidence levels, that may for example score the linking, matching, mapping, and/or correlating with a respective confidence level.

Linking the ultrasound image data with the other image data based on the deformability properties may comprise: Linking a simulated deformed medium/a simulation of a deformation of the medium, for example a simulation of a deformation of the medium compared to the medium as associated with the ultrasound image data, with the other image data. The simulation for example may be based on the ultrasound image data.

Linking the ultrasound image data with the other image data based on the deformability properties may comprise: Linking the ultrasound image data with a simulated deformed medium/a simulation of a deformation of the medium, for example a simulation of a deformation of the medium compared to the medium as associated with the other image data. The simulation for example may be based on the other image data.

Linking the ultrasound image data with the other image data may comprise: Identifying ultrasound image and other image data that each represent the same and/or a similar portion of the medium and/or the same and/or a similar region in the medium.

The operation (120) of linking the ultrasound image data with the other image data based on the deformability properties may comprise an optional operation of simulating a deformed medium based on the deformability properties. A deformed medium may be simulated such that the simulated deformed medium matches with the deformation state of the medium as associated with the other image data.

The simulated deformed medium may be simulated based on the deformability properties and/or based on the ultrasound image data and/or based on the other image data.

Simulating a deformed medium may comprise building a model of the medium, for example a parametric model of the medium, for example a 3D parametric model. The parametric model may be based on the deformability properties of the medium. Simulating a deformed medium may be based on the deformability properties of the medium.

Linking (120) the ultrasound image data with the other image data based on the deformability properties may further be based on the simulated deformed medium.

The operation (120) of linking the ultrasound image data with the other image data based on the deformability properties may comprise an optional operation of determining a first subset of the ultrasound image data, and/or a plurality of first subsets of the ultrasound image data.

The operation (120) of linking the ultrasound image data with the other image data based on the deformability properties may comprise an optional operation of determining a second subset of the other image data, and/or a plurality of second subsets of the other image data.

The first subset of the ultrasound image data may be associated with (for example correspond to) a first region in the medium, and the second subset of the other image data may be associated with (for example correspond to) a second region in the medium/second portion of the medium.

The first portion of the medium and the second portion of the medium may correspond to spatial regions in the medium. The first portion of the medium and the second portion of the medium may correspond to lesion(s) in the medium. The first portion of the medium and the second portion of the medium may be determined based on manual input and/or based on using a predefined computer-implemented algorithm, for example a regular automated algorithm and/or an AI-based algorithm, for example it may involve using a neural network, a convolutional neural network or the like. For example, a tumor recognition technique may be used. The first portion of the medium and the second portion of the medium may be determined based on pattern recognition schemes.

The first region in the medium/the first portion of the medium may overlap with and/or may be identical to the second region in the medium/the second portion of the medium. In particular, the first region in the medium/the first portion of the medium and the second region in the medium/the second portion of the medium may comprise similar or quasi identical tissue.

The operation (120) of linking the ultrasound image data with the other image data based on the deformability properties may comprise an optional operation of linking a second subset with a first subset, optionally based on the simulated deformed medium.

Linking the second subset with the first subset may for example allow to link a lesion identified in a mammography and/or a tomosynthesis examination with a lesion identified in an ultrasound examination. The first subset and/or the second subset may for example comprise lesions and/or conspicuous tissue. The lesion identified in the ultrasound image data and the lesion identified in the other image data for example may be the same lesions. Linking the second subset with the first subset may for example allow to link the location of a lesion identified in a mammography and/or a tomosynthesis examination with the location of a lesion identified in an ultrasound examination.

Determining the first subset and/or determining the second subset and/or linking the second subset with the first subset may be based on tracking schemes, for example contour tracking, Kalman filtering, particle filtering, learning schemes, for example deep learning, and/or a block matching algorithm.

Linking the ultrasound image data with the other image data may be based on the simulated deformed medium. Linking the second subset with the first subset may be based on the simulated deformed medium, for example linking the second subset with the first subset may comprise linking the second subset with a simulated deformed first subset and/or a first subset in a simulated deformed medium. Linking the second subset with the first subset may be based on the simulated deformed medium, for example linking the second subset with the first subset may comprise linking a deformed second subset and/or a second subset in a simulated deformed medium with the first subset.

In an optional operation (130), a linking confidence, e.g. a linking confidence parameter and/or level of linking the ultrasound image data with the other image data is evaluated, i.e. determined.

Determining a linking confidence level of linking the ultrasound image data with the other image data may be based on at least one of: the deformability properties, the deformability properties confidence.

The linking confidence level may be for example a confidence index.

Determining (130) a linking confidence of linking the ultrasound image data with the other image data may comprise the optional operation of determining a deformability properties confidence.

The deformability properties confidence for example may be a measure for the quality and/or the confidence of the estimation of the deformability properties. The deformability properties confidence for example may be error bars/uncertainty bars at pre-defined confidence levels. A lower deformability properties confidence may for example lead to a lower linking confidence. Further, it for example may be easier to link the ultrasound image data with the other image data if deformability properties are within specific value ranges. In this way for example, a linking confidence may be based on the deformability properties/may be impacted by the deformability properties.

The deformability properties confidence for example may be related to differences between the simulated deformed medium and the medium as represented by/associated with and/or reconstructed from the other image data. The deformability properties confidence for example may be determined based on differences between the simulated deformed medium and the medium as represented by and/or reconstructed from the other image data.

Determining a deformability properties confidence may be performed using a computer-implemented algorithm.

Determining (130) a linking confidence of linking the ultrasound image data with the other image data may comprise the optional operation of determining a linking confidence of linking the second subset with the first subset, and/or may be based on determining the linking confidence of linking the second subset with the first subset.

Determining a linking confidence of linking the second subset with the first subset may be based on at least one of: a spatial distance of the first portion and of/between the second portion, a spatial distance of the first portion in a simulated deformed medium and/between the second portion, a spatial overlap between the first portion and the second portion, a spatial overlap of the first portion in a simulated deformed medium and the second portion, a contour and/or a shape and/or a size of the first and/or the second portion, a contour and/or a shape and/or a size of the first portion in a simulated deformed medium and/or of the second portion, the deformability properties, the deformability properties confidence.

For example, if contours and/or shapes and/or sizes of the first portion and the second portion are very similar, the linking confidence may be higher. Determining a linking confidence of linking the second subset with the first subset may be based on a Bayesian method and/or a conditional method, and for example based on the deformability properties confidence and/or differences between the simulated deformed medium and the medium as represented by and/or reconstructed from the other image data.

Simulating a deformed medium and/or determining a first subset and/or determining a second subset and/or linking the first subset with the second subset and/or determining a linking confidence of linking the ultrasound image data with the other image data and/or determining a linking confidence of linking the second subset with the first subset may be performed using a computer-implemented algorithm.

Determining a deformability properties confidence may be performed using a computer-implemented algorithm.

The computer-implemented algorithm for example may be AI-based, it may comprise use of learning schemes, for example deep learning and/or machine learning, and/or an artificial intelligence and/or a neural network and/or a convolutional neural network and/or the like. The computer-implemented algorithm may further be based on manual input by a user and/or may be performed in an automated way. The computer-implemented algorithm may be for example Kernel-based, it may involve for example contour tracking, Kalman filtering, particle filtering. The computer-implemented algorithm may be for example automated.

The computer-implemented algorithm may comprise applying tracking schemes for linking the first subset with the second subset and/or for determining a linking confidence. The applied tracking schemes may involve contour tracking, Kalman filtering, particle filtering, learning schemes, for example deep learning.

Also a plurality of first subsets associated with/corresponding to a plurality of first portions of the medium/a plurality of first regions in the medium and/or a plurality of second subsets associated with/corresponding to a plurality of second portions of the medium/a plurality of second regions in the medium may be determined and may be linked with each other, for example using a loss function, and for example corresponding linking confidences may be determined. For example, several loops of linking first subsets with second subsets may be performed and matrices with linking confidences may be built.

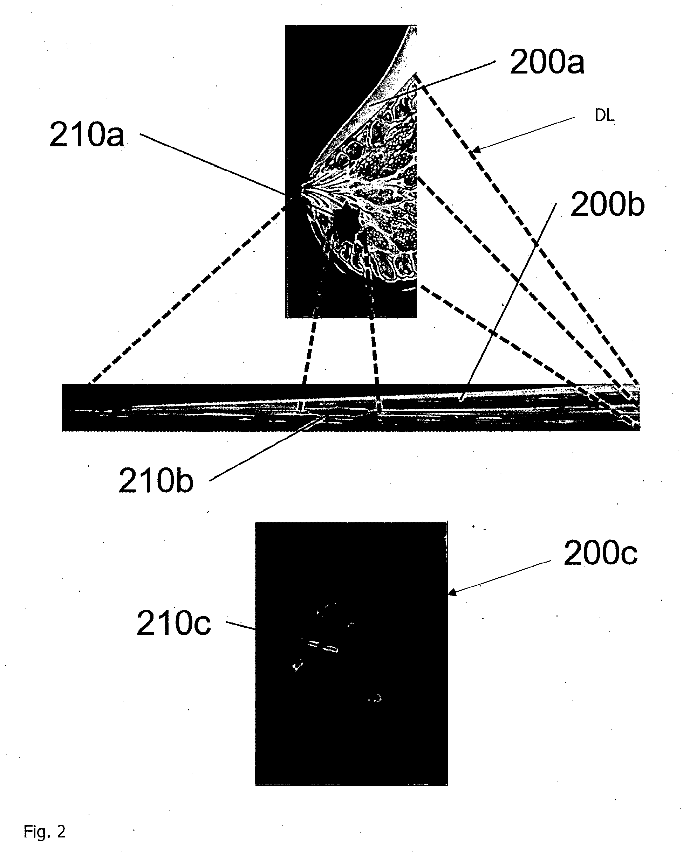

Fig. 2 illustrates an exemplary relation between a parametric model of the medium, a simulated deformed medium, and other image data.

A model 200a of the medium, i.e. a human breast in this example, is shown. The model 200a may be a parametric model and/or a geometrical/physical model. The model 200a may be based on the ultrasound image data, for example 3D ultrasound image data. The medium comprises a first portion. The portion of the parametric model representing/associated with the first portion is denoted with 210a. The model may be based on deformability properties of the medium. Deformability properties are estimated based on the ultrasound data.

The first portion may be distinguished from the surrounding medium based on at least one of: elasticity properties of the medium, non-linear elasticity properties of the medium, transparency properties, reflection properties, viscoelastic properties, an image segmentation and/or classification method using a predefined computer-implemented algorithm.

The first portion of the medium may comprise at least one of: a tumor, a lesion, suspicious tissue, a tumor candidate, a marker, a breast cancer. The first portion may be a lesion, for example a suspicious lesion.

A simulated deformation of the model, for example of the parametric model, and/or of the medium is denoted with 200b. The portion of the simulated deformed medium representing/associated with the first portion is denoted with 210b. Due to the deformation, the shape, contour and size of the of the portion 210b is changed compared to the portion 210a (cf. also the dashed lines DL which schematically indicate the deformation). Due to the deformation, the shape, contour and size of the medium 200b is changed compared to the medium 200a. The relation between the model 200a and the simulated deformation 200b is also indicated by the dotted lines in the figure.

The simulated deformation may match with the deformation state of the medium as associated with/represented by the other image data, for example the mammography and/or tomography image data.

Other image data, for example tomosynthesis image data, are denoted with 200c. The portion of the other image data/the tomosynthesis data representing/associated with the first portion is denoted with 210c.

The present disclosure may allow to link the ultrasound image data with other image data, for example with the tomosynthesis data 210c, based on the deformability properties. The present disclosure may allow to link the portions 210a and 210c, for example based on the portion 210b, for example based on the deformability properties.

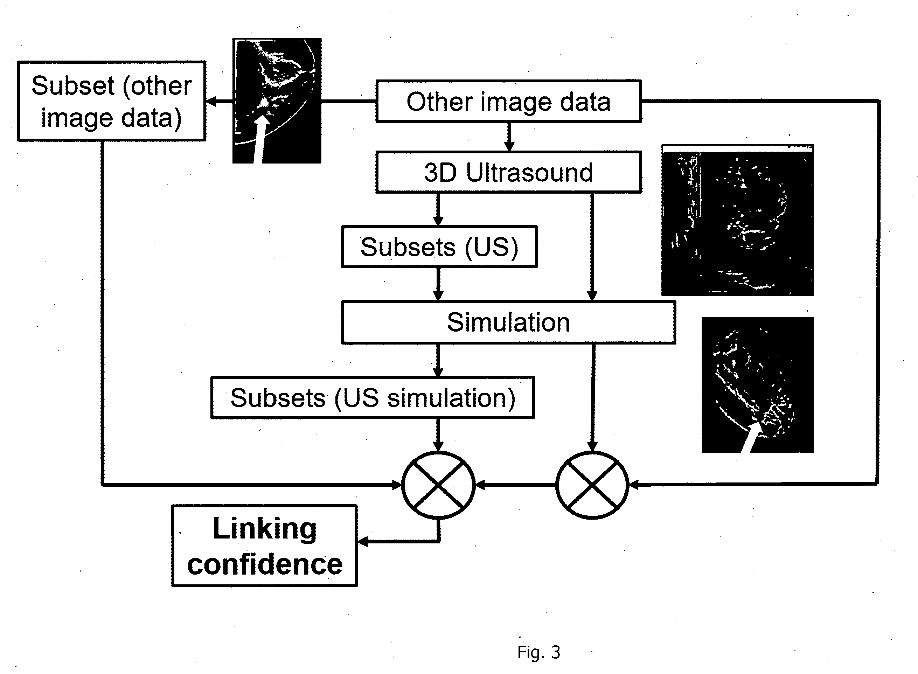

Figure 3 shows another exemplary embodiment of a method according to embodiments of the present disclosure.

A second subset of the other image data, for example mammography image data and/or tomosynthesis data, may be determined (indicated by the white arrow in the upper left picture). The second subset may for example correspond to a suspicious lesion of the medium. A plurality of second subsets of the other image data may be determined.

Ultrasound imaging may be performed, for example 3D ultrasound imaging. Corresponding ultrasound image data are shown in the upper right picture. Based on the ultrasound image data, deformability properties are estimated.

One or more first subsets of the ultrasound image data may be determined. The one or more first subset may for example correspond to one or more suspicious lesion of the medium.

A deformed medium may be simulated based on the deformability properties. The simulation may be such that the simulated deformed medium matches with the deformation state of the medium as associated with the other image data, for example the mammography image data and/or tomosynthesis data.

One or more simulated deformed first subsets may be determined in the deformed simulated medium.

The first subsets may be linked with the second subset, for example based on the deformability properties. The simulated deformed first subsets may be linked with the second subset.

The ultrasound image data are linked with the other image data based on the deformability properties, for example based on the simulated deformed medium.

A linking confidence of linking the ultrasound image data with the other image data may be determined. The linking confidence for example may be determined, based on the deformability properties and/or a linking confidence of linking the second subset with the first subset.

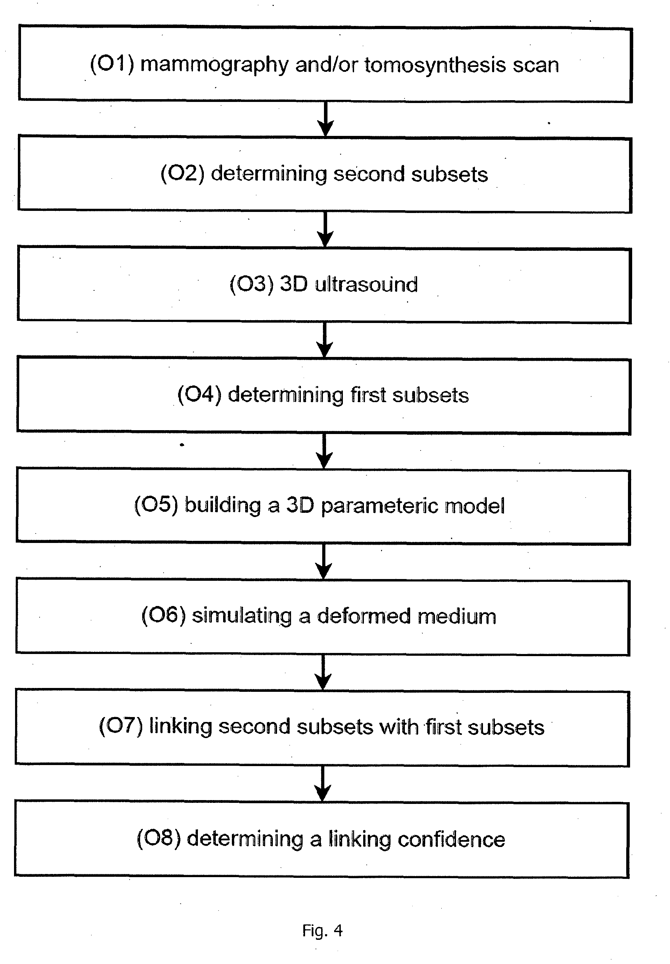

Fig. 4 shows yet another exemplary embodiment of a method according to embodiments of the present disclosure.

During operation 1 (O1), a mammography and/or a tomosynthesis scan may be performed and other image data may be obtained.

During operation 2 (02), one or more second subsets of the other image data may be determined.

During operation 3 (03), an ultrasound, for example a 3D ultrasound, of the medium, for example of a human breast, may be performed, and ultrasound image data may be obtained.

During operation 4 (04), one or more first subsets of the ultrasound image data may be determined.

During operation 5 (05), a parametric model, for example a 3D parametric model, may be built. The 3D parametric model may comprise/may be based on deformability properties. Deformability properties are estimated based on the ultrasound image data. A deformability properties confidence may be determined.

During operation 6 (06), a deformed medium may be simulated. The simulation may be based on the deformability properties and/or based on the model. The simulation may be such that the simulated deformed medium matches with the deformation state of the medium as associated with the other image data.

During operation 7 (07), the ultrasound image data are linked with the other image data based on the deformability properties. Linking the ultrasound image data with the other image data may comprise: Linking the second subset/the one or more second subsets with the one or more first subsets and/or linking the second subset/ the one or more second subsets with the one or more simulated deformed first subsets. Linking the one or more second subsets with the one or more first subsets may comprise: Determining a linking confidence of linking the one or more second subsets with the one or more first subsets.

During operation 8 (08), a linking confidence may be determining. A linking confidence may be determined for example based on the deformability properties and/or the deformability properties confidence and/or the linking confidence of linking the one or more second subsets with the one or more first subsets.

Examples of the present disclosure may allow to link ultrasound image data with other image data in reliable, robust, reproducible, precise, accurate, and/or fast way.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "associated with" and "represented by" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

In summary the method according the present disclosure as described above provides a method and system of linking ultrasound image data associated with a medium with other image data associated with the medium, which advantageously is reliable and robust. Further, due to the increased preciseness a decreased variance and thus an increased reproducibility can advantageously be achieved.

## Claims

1. A method (100) of linking ultrasound image data associated with a medium with other image data associated with the medium, comprising:
estimating (110) deformability properties of the medium based on the ultrasound data, and
linking (120) the ultrasound image data with the other image data based on the deformability properties.

2. The method according to claim 1, wherein the other image data comprise at least one of:
• image data originating from another source than an ultrasound imaging source,
• X-ray image data,
• mammography and/or tomosynthesis and/or tomography image data,
• Magnetic Resonance (MR) image data,
• image data originating from an optical imaging device, and
• other ultrasound image data.

3. The method according to claim 1 or 2,
wherein the ultrasound data comprise 3D ultrasound image data, and/or
wherein the ultrasound data comprise at least one of:
• shear wave elasticity (SWE) data,
• non-linear shear wave elasticity (NL-SWE) data,
• B-mode data.

4. The method according to any of the preceding claims, wherein the medium as associated with the other image data is in a different deformation state compared to the medium as associated with the ultrasound image data.

5. The method according to any of the preceding claims, wherein linking (120) the ultrasound image data with the other image data comprises:
linking the ultrasound image data with third image data, and linking the other image data with the third image data.

6. The method of any of the preceding claims, wherein the medium comprises human breast tissue.

7. The method according to any of the preceding claims, wherein estimating (110) deformability properties comprises estimating at least one of:
• elasticity properties of the medium,
• non-linear elasticity properties of the medium,
• stiffness properties of the medium,
• geometrical properties of the medium,
• structural and/or granularity properties of the medium,
• density properties of the medium, and
• absorption properties of the medium.

8. The method according to any of the preceding claims, wherein deformability properties are estimated based on at least one of the following methods:
• Finite Element modelling,
• Finite Element modelling using boundaries,
• Modelling using Active Cubes,
• Boundary Element Modelling, and
• a predefined registration method.

9. The method according to any of the preceding claims, wherein linking (120) the ultrasound image data with the other image data comprises:
simulating a deformed medium based on the deformability properties such that the simulated deformed medium matches with the deformation state of the medium as associated with the other image data.

10. The method according to the preceding claims, wherein simulating the deformed medium is further based on the other image data and/or simulating the deformed medium uses the other image data in a control loop.

11. The method according to any of the preceding claims, wherein linking (120) the ultrasound image data with the other image data comprises:
determining a first subset of the ultrasound image data associated with a first portion of the medium,
determining a second subset of the other image data associated with a second portion of the medium, and
linking the second subset with the first subset, optionally based on the simulated deformed medium.

12. The method according to the preceding claim, wherein the first portion of the medium is distinguished from the surrounding medium based on at least one of:
elasticity properties of the medium,
non-linear elasticity properties of the medium,
an image segmentation and/or classification method using a predefined computer-implemented algorithm.

13. The method according to claim 11 or 12, wherein
the second portion of the medium is distinguished from the surrounding medium based on an image segmentation and/or classification method using a predefined computer-implemented algorithm.

14. The method according to any of the preceding claims, further comprising:
determining a deformability properties confidence, and
determining (130) a linking confidence of linking the ultrasound image data with the other image data,
wherein determining a linking confidence is based on at least one of:
• the deformability properties, and
• the deformability properties confidence. ,

15. The method according to the preceding claim,
wherein the method further comprises determining a linking confidence of linking the second subset with the first subset based on at least one of:
• a spatial distance of the first portion and of the second portion,
• a spatial distance of the first portion in a simulated deformed medium and the second portion,
• a spatial overlap between the first portion and the second portion,
• a spatial overlap of the first portion in a simulated deformed medium and the second portion,
• a contour of the first and/or the second portion,
• a contour of the first portion in a simulated deformed medium and/or of the second portion,
• the deformability properties, and
• the deformability properties confidence.

16. The method according to the preceding claim,
wherein determining the linking confidence of linking the ultrasound image data with the other image data is based on determining the linking confidence of linking the second subset with the first subset.

17. The method according to any of claims 9 to 16, wherein the operations of simulating a deformed medium and/or determining a first subset and/or determining a second subset and/or linking the second subset with the first subset and/or determining the linking confidence of linking the ultrasound image data with the other image data and/or determining the linking confidence of linking the second subset with the first subset are performed using a computer-implemented algorithm.

18. A computer program comprising computer-readable instructions which when executed by a data processing system cause the data processing system to carry out the method according to any one of preceding claims.

19. A system for linking ultrasound image data associated with a medium with other image data associated with the medium, the system comprises a processing unit configured to:
estimate deformability properties of the medium based on the ultrasound data, and
link the ultrasound image data with the other image data based on the deformability properties.
